(19) Europäisches Patentamt
European Patent Office
Office européen des brevets

(11) Veröffentlichungsnummer: **0 021 077**
**B1**

(12) # EUROPÄISCHE PATENTSCHRIFT

(45) Veröffentlichungstag der Patentschrift: **23.03.83**

(21) Anmeldenummer: **80102944.8**

(22) Anmeldetag: **27.05.80**

(51) Int. Cl.³: **C 07 C 149/437,** C 08 G 18/77, C 08 G 18/78, C 08 G 18/30, C 08 G 18/10

(54) **Gegebenenfalls in NCO-Präpolymeren gelöste, Harnstoffgruppen aufweisende Diisocyanate, ein Verfahren zu ihrer Herstellung, sowie ihre Verwendung zur Herstellung von Polyurethankunststoffen.**

(30) Priorität: **06.06.79 DE 2922966**

(43) Veröffentlichungstag der Anmeldung: **07.01.81 Patentblatt 81/1**

(45) Bekanntmachung des Hinweises auf die Patenterteilung: **23.03.83 Patentblatt 83/12**

(84) Benannte Vertragsstaaten: **AT BE DE FR GB IT NL**

(56) Entgegenhaltungen:
**DE-A-2 015 159**
**DE-C-1 020 327**
**GB-A-1 131 808**

(73) Patentinhaber: **BAYER AG, Zentralbereich Patente, Marken und Lizenzen, D-5090 Leverkusen 1, Bayerwerk (DE)**

(72) Erfinder: **Schwindt, Jürgen, Dr., Kleist-Platz 4, D-5090 Leverkusen (DE)**
Erfinder: **Grögler, Gerhard, Dr., von Diergardt-Strasse 46, D-5090 Leverkusen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 021 077 B1

# Gegebenenfalls in NCO-Präpolymeren gelöste, Harnstoffgruppen aufweisende Diisocyanate, ein Verfahren zu ihrer Herstellung, sowie ihre Verwendung zur Herstellung von Polyurethankunststoffen

Die vorliegende Erfindung betrifft neue, bei Raumtemperatur flüssige oder durch einfaches Erhitzen auf maximal 80°C verflüssigbare, gegebenenfalls als Lösung in NCO-Präpolymeren vorliegende, Harnstoffgruppen aufweisende Diisocyanate, ein Verfahren zu ihrer Herstellung durch Umsetzung spezieller Ausgangsdiisocyanate mit Wasser, sowie ihre Verwendung als Aufbaukomponente bei der Herstellung von Polyurethankunststoffen.

Es ist seit langem bekannt, daß die Reaktion von Wasser mit Monoisocyanaten zu substituierten Harnstoffen und mit Polyisocyanaten zu hochmolekularen Polyharnstoffen führt. Isocyanatgruppen enthaltende harzartige Polyharnstoffe kann man gemäß US-PS 2 597 025 dann erhalten, wenn in geeigneten Lösungsmitteln pro Mol aromatischem Diisocyanat 0,3 bis 0,6 Mol $H_2O$ verwendet werden. Darüber hinaus ist bekannt geworden, daß aromatische Diisocyanate mit $H_2O$ auch selektiv zu niedermolekularen Diisocyanatoharnstoffen umgesetzt werden können. So erhält man gemäß US-PS 2 757 184, US-PS 2 757 185 und US-PS 3 906 019 aus 2,4-Diisocyanatotoluol mit $H_2O$ unter geeigneten Reaktionsbedingungen den entsprechenden Bis-(3-isocynatotolyl)-harnstoff.

Auch die analoge Reaktion von 2,6-Diisocyanatotoluol zu dem 1,3-Bis-(3-isocyanatotolyl)-harnstoff ist bekannt (US-PS 3 906 019, US-PS 2 902 474).

Alle diese Verfahren werden in Lösungsmittel durchgeführt. In diesen Lösungsmitteln müssen die Diisocyanate gut und das zugesetzte $H_2O$ zumindest teilweise löslich sein. Es darf keine polymerisierende Wirkung auf das Isocyanat ausüben und muß frei von gegenüber NCO-Gruppen reagierenden funktionellen Gruppen sein.

Der Hauptnachteil dieser Harnstoffgruppen aufweisenden Diisocyanate des Standes der Technik ist darin zu sehen, daß sie bei dem genannten Verfahren zu ihrer Herstellung stets als nicht oder nur bei sehr hohen Temperaturen schmelzbare Festkörper der unterschiedlichsten Korngröße anfallen, so daß sie für ihre weitere Verarbeitung, beispielsweise als Ausgangsmaterial für die Herstellung von Polyurethanen nach ihrer Isolierung durch Filtration und Entfernung von anhaftenden Lösungsmittelmengen im Vakuum durch aufwendige Mahlvorgänge in eine feinverteilte Form gebracht werden müssen. Darüber hinaus werden bei der Umsetzung dieser Harnstoffgruppen aufweisenden Diisocyanate des Standes der Technik wegen ihres hohen Schmelzpunkts und ihrer Schwerlöslichkeit inhomogene Produkte erhalten, die im übrigen oft ein schlechtes mechanisches Eigenschaftsniveau aufweisen. Infolge des festen Aggregatzustandes und der Schwerlöslichkeit der Harnstoffgruppen aufweisenden Diisocyanate des Standes der Technik sind bei ihrer Verarbeitung genaue Äquivalentverhältnisse zwischen Isocyanatgruppen und gegenüber Isocyanatgruppen reaktionsfähigen Gruppen nur schwer einzuhalten, da die Harnstoffdiisocyanatteilchen oft nur an der Oberfläche abreagieren, wobei eine Hülle um nicht abreagiertes Harnstoffdiisocyanat gebildet wird.

Andererseits stellen Harnstoffgruppen aufweisende Diisocyanate wertvolle Ausgangsmaterialien zur Herstellung von Polyurethankunststoffen dar, da die durch ihre Verwendung als Aufbaukomponente eingebauten Harnstoffsegmente oft zu einer Verbesserung der mechanischen Eigenschaften des Kunststoffs führen.

Es war daher die Aufgabe der vorliegenden Erfindung, neue Harnstoffdiisocyanate und ein Verfahren zu ihrer Herstellung zur Verfügung zu stellen, die einerseits wegen ihrer Harnstoff-Segmente die Herstellung von Polyurethankunststoffen mit verbesserten mechanischen Eigenschaften gestatten, und die andererseits nicht mehr mit den geschilderten Nachteilen der Verbindungen bzw. Verfahren des Standes der Technik behaftet sind.

Diese Aufgabe konnte überraschenderweise dadurch gelöst werden, daß man bestimmte, nachstehend näher beschriebene, Schwefel aufweisende Diisocyanate mit Wasser zu den entsprechenden Harnstoffgruppen aufweisenden Diisocyanaten umsetzt.

Gegenstand der vorliegenden Erfindung sind bei Raumtemperatur flüssige oder durch Erhitzen auf maximal 80°C verflüssigbare, gegebenenfalls als bei Raumtemperatur flüssige oder durch Erhitzen auf maximal 80°C verflüssigbare, 5 bis 50 gew.-%ige Lösung in bei Raumtemperatur flüssigen oder durch Erhitzen auf maximal 80°C verflüssigbaren NCO-Präpolymeren der Formel

$$D-(OCO-NH-A-NCO)_n$$

vorliegende, Harnstoffgruppen aufweisende Diisocyanate der Formel

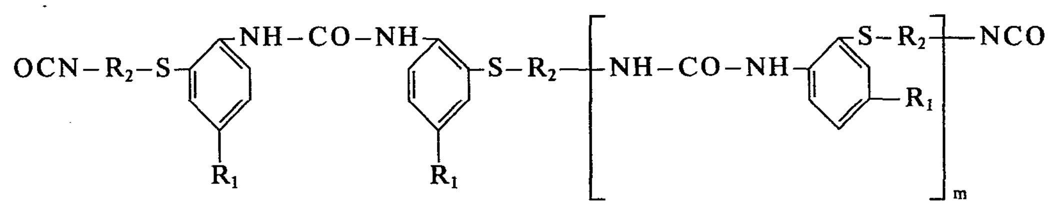

wobei

m für O oder eine ganze oder (im statistischen Mittel) gebrochene Zahl von 0 bis 3 steht,
n für eine ganze oder (im statistischen Mittel) gebrochene Zahl von 2 bis 4 steht,
A für einen Rest steht, wie er durch Entfernung der Isocyanatgruppen aus einem organischen Diisocyanat erhalten wird,
D für einen Rest steht, wie er durch Entfernung der Hydroxylgruppen aus einer n-funktionellen Polyhydroxylverbindung des Molekulargewichtsbereichs 500 bis 8000, bzw. durch Entfernung der Hydroxylgruppen aus einem Gemisch derartiger Polyhydroxylverbindungen erhalten wird,
$R_1$ Wasserstoff, eine Alkylgruppe mit 1 bis 4 Kohlenstoffatomen oder eine Alkylthiogruppe mit 1 bis 4 Kohlenstoffatomen bedeutet,
$R_2$ für einen, gegebenenfalls durch Alkylgruppen mit 1 bis 4 Kohlenstoffatomen oder durch eine Alkylthiogruppe mit 1 bis 4 Kohlenstoffatomen substituierten Phenylenrest oder einen linearen oder verzweigten aliphatischen Kohlenwasserstoffrest mit 2 bis 12 Kohlenstoffatomen steht, wobei zwischen dem Stickstoffatom und dem Schwefelatom mindestens 2 Kohlenstoffatome angeordnet sind.

Gegenstand der vorliegenden Erfindung ist auch ein Verfahren zur Herstellung dieser neuen Diisocyanate, welches dadurch gekennzeichnet ist, daß man Diisocyanate der Formel

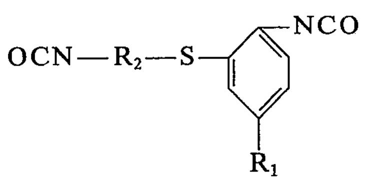

mit 0,4 bis 0,8 Mol pro Mol Diisocyanat Wasser oder einer entsprechenden Menge einer wasserabspaltenden Verbindung zur Reaktion bringt und gegebenenfalls das so erhaltene Harnstoffgruppen aufweisende Diisocyanat anschließend in 50 bis 95 Gew.-%, bezogen auf Gesamtgemisch, eines NCO-Präpolymeren der obengenannten Art löst.

Gegenstand der vorliegenden Erfindung ist schließlich auch die Verwendung der neuen, gegebenenfalls in NCO-Präpolymeren gelösten Diisocyanate als Aufbaukomponente bei der Herstellung von Polyurethankunststoffen nach dem Isocyanat-Polyadditionsverfahren.

In den genannten Formeln haben die variablen m,n,A,D,$R_1$ und $R_2$ die genannte Bedeutung. Vorzugsweise stehen hier und auch nachstehend

m für O,
n für 2,
A für einen aliphatischen Kohlenwasserstoffrest mit 6 bis 12, insbesondere 6 Kohlenstoffatomen, wobei zwischen den beiden Stickstoffatomen mindestens 6 Kohlenstoffatome angeordnet sind, einen cycloaliphatischen Kohlenwasserstoffrest mit 4 bis 15 Kohlenstoffatomen, einen aromatischen Kohlenwasserstoffrest mit 6 bis 15 Kohlenstoffatomen, einen Xylylenrest, oder einen Rest der Formel

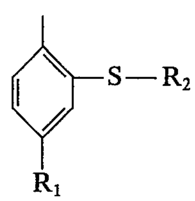

$R_1$ für Wasserstoff und
$R_2$ für einen Polymethylenrest mit 2 bis 6 Kohlenstoffatomen oder einen 1,4-Phenylenrest.

Die Herstellung der für das erfindungsgemäße Verfahren als Ausgangsmaterial einzusetzenden Diisocyanate erfolgt entsprechend der Offenbarung der deutschen Patentanmeldung P 2 916 135.1 durch Phosgenierung von Diaminen der Formel

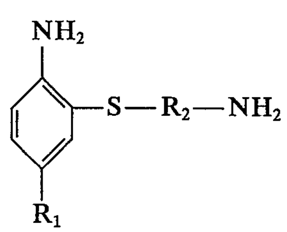

Die hierzu als Ausgangsmaterialien einzusetzenden, Thioethergruppen aufweisenden Diamine sind nach bekannten Methoden des Standes der Technik zugänglich.

Die Thioethergruppen aufweisenden Ausgangsamine mit einer aliphatisch gebundenen Aminogruppe sind beispielsweise in Analogie zum Verfahren der DE-OS 2 734 575 durch Umsetzung der entsprechenden Natriumamino-thiophenolate der Formel

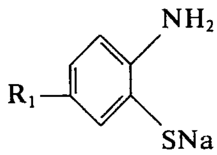

mit entsprechenden Chloraminen derr Formel

$$Cl—R_2—NH_2$$

zugänglich, wobei die genannten Natriumthiophenolate auf einfache Weise durch alkalische Verseifung der entsprechenden Benzothiazole erhalten werden können.

Die ebenfalls einsetzbaren, ausschließlich aromatisch gebundene Aminogruppen und Thioethergruppen aufweisenden Diamine können beispielsweise durch Umsetzung der bereits genannten, o-aminosubstituierten Natrium-thiophenolate mit den entsprechenden p-Nitro-chlorbenzolen der Formel

$$Cl—R_2—NO_2$$

zu den entsprechenden eine Amino- und eine Nitrogruppe aufweisenden Zwischenstufen umgesetzt werden, worauf sich eine Hydrierung der Nitrogruppe zur Aminogruppe, beispielsweise mittels Zink/Chlorwasserstoff oder unter Verwendung von Ranney-Nickel als Katalysator, anschließt. Die Herstellung der genannten Zwischenstufe wird in Prinzip beispielsweise in J. Chem. Soc. London 1930, 180 ff beschrieben.

Eine weitere Methode zur Herstellung von 2 aromatisch gebundene Aminogruppen aufweisenden Thioethern besteht beispielsweise in der Umsetzung der zuletzt genannten p-Nitrochlorbenzole mit Natriumsulfid zu den entsprechenden p-Amino-thiophenolaten der Formel

$$H_2N—R_2—SNa$$

und deren anschließender Kondensation mit o-Chlornitrobenzolen der Formel

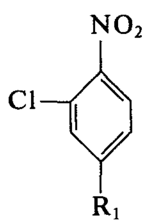

worauf sich auch hier wieder eine Hydrierung der noch vorliegenden Nitrogruppe anschließt. Die Herstellung der eine Aminogruppe und eine Nitrogruppe aufweisenden Zwischenstufe nach diesem Prinzip ist beispielsweise in J. Chem. Soc. London 1930, 180 beschrieben.

Schließlich sind die Thioethergruppen aufweisenden Diamine mit 2 aromatisch gebundenen Aminogruppen auch durch Umsetzung der entsprechenden o-Nitro-thiophenolate mit den entsprechenden p-Nitrochlorbenzolen bzw. durch Umsetzung der entsprechenden p-Nitro-thiophenolate mit den entsprechenden o-Nitro-chlorbenzolen zu der 2 Nitrogruppen aufweisenden Zwischenstufe und deren anschließende Hydrierung zugänglich. Die Herstellung der bei dieser Methode vorliegenden Zwischenstufe ist beispielsweise in Journal of the American Chemical Society 45, 1399 ff beschrieben.

Geeignete Diamine sind beispielsweise 2-(2′-Aminoethylthio)-anilin, 2-(6′-Aminohexylthio)-anilin, 2-(12′-Aminododecylthio)-anilin, 2-(2′-Aminoethylthio)-5-methoxyanilin, 2-(2′-Aminoethylthio)-5-chlor-anilin, 2-(6′-Aminohexylthio)-5-ethylsulfono-anilin, 2,4′-Diaminodiphenylsulfid oder 2,4′-Diamino-3′-ethylthio-diphenylsulfid.

Die Phosgenierung der beispielhaft genannten Diamine zu den entsprechenden Diisocyanaten erfolgt nach an sich bekannten Methoden, vorzugsweise unter Mitverwendung eines geeigneten Hilfslösungsmittels wie z. B. Chlorbenzol bei −20 bis 130° C.

Geeignete Phosgenierverfahren sind beispielsweise in High Polymers XVI »Polyurethanes«, Chemistry and Technology, Part I, Interscience Publishers, New York, London 1962, Seiten 17 ff beschrieben.

Die bei dieser Phosgenierungsreaktion anfallenden Diisocyanate entsprechen naturgemäß bezüglich ihrer Konstitution den beispielhaft genannten, als Ausgangsmaterial eingesetzten Diaminen.

Die Durchführung des erfindungsgemäßen Verfahrens erfolgt im allgemeinen nach einer der beiden nachstehenden Varianten:

1. Die Schwefel enthaltenden Ausgangsdiisocyanate werden als 10- bis 90gew.-%ige, vorzugsweise 30- bis 70gew.-%ige Lösung in einem inerten Lösungsmittel bei 25 bis 100° C, vorzugsweise 40 bis 70° C mit 0,4 bis 0,8 Mol, vorzugsweise 0,5 bis 0,6 Mol Wasser oder einer entsprechenden Menge einer Wasser abspaltenden Verbindung pro Mol Ausgangsdiisocyanat umgesetzt. Hierzu wird die Lösung des Ausgangsdiisocyanats vorzugsweise vorgelegt und das Wasser bzw. die Wasser abspaltende Verbindung der vorgelegten Lösung zudosiert. Der Reaktionsverlauf kann durch Bestimmung des Gasvolumens (Kohlendioxid-Entwicklung) leicht verfolgt und kontrolliert werden. Nach beendeter Umsetzung kann das Lösungsmittel beispielsweise destillativ entfernt werden. Die erfindungsgemäßen Verbindungen fallen dann als Destillationsrückstand an.
   Geeignete inerte Lösungsmittel sind beispielsweise Aceton, Methylethylketon, Methylisobutylketon, Dioxan, Cyclohexanon, Acetessigester oder Acetylaceton.
   Geeignete Wasser abspaltende Verbindungen sind beispielsweise Ameisensäure, tert.-Alkohole wie z. B. tert.-Butanol, kristallwasserhaltige organische oder anorganische Verbindungen wie Pinakolhexahydrat, Chloralhydrat oder Natriumsulfat-Decahydrat. Vorzugsweise wird Wasser in flüssiger Form eingesetzt.
2. Das Schwefel enthaltende Ausgangsdiisocyanat wird mit einem bei Raumtemperatur flüssigen oder durch Erhitzen auf maximal 80° C schmelzbaren NCO-Präpolymer der Formel

   $$D{-}\!\!(OCO{-}NH{-}A{-}NCO)_n$$

   vermischt, wobei die Mengenverhältnisse so gewählt werden, daß im Gesamtgemisch ca. 5 bis 50, vorzugsweise 5 bis 25 Gew.-% des Ausgangsdiisocyanats vorliegen. Anschließend wird dem so erhaltenen Gemisch Wasser oder eine Wasser abspaltende Verbindung der beispielhaft genannten Art unter intensivem Durchmischen in einer solchen Menge zugeführt, daß auf jedes Grammäquivalent an Isocyanatgruppen des Schwefel enthaltenden Diisocyanats 0,4 bis 0,8, vorzugsweise 0,5 bis 0,6 Mol Wasser entfallen. Das Reaktionsgemisch wird hierbei innerhalb der o. g. Temperaturbereiche gehalten.
   Auch hier kann der Verlauf der Umsetzung an der Menge des freigesetzten Kohlendioxids volumetrisch verfolgt werden. Es werden auf diese Weise unmittelbar Lösungen der erfindungsgemäßen Diisocyanate in den NCO-Präpolymeren erhalten.

Zur Herstellung der Isocyanat-Präpolymeren geeignete Polyole der Formel

$$D(OH)_n$$

sind 2 bis 4, vorzugsweise 2 Hydroxylgruppen aufweisende Polyester, Polyether, Polythioether, Polyacetale, Polycarbonate oder Polyesteramide des Molekulargewichtsbereichs 500 bis 8000, vorzugsweise 1000 bis 3000, wie sie für die Herstellung von homogenen oder geschäumten Polyurethanen an sich bekannt sind. Vorzugsweise gelangen die entsprechenden Polyester- bzw. Polyetherpolyole zum Einsatz.

Die in Frage kommenden Hydroxylgruppen aufweisenden Polyester sind z. B. Umsetzungsprodukte von mehrwertigen, vorzugsweise zweiwertigen und gegebenenfalls zusätzlich dreiwertigen Alkoholen mit mehrwertigen, vorzugsweise zweiwertigen, Carbonsäuren. Anstelle der freien Polycarbonsäuren können auch die entsprechenden Polycarbonsäureanhydride oder entsprechende Polycarbonsäureester von niedrigen Alkoholen oder deren Gemische zur Herstellung der Polyester verwendet werden. Die Polycarbonsäuren können aliphatischer, cycloaliphatischer, aromatischer und/oder heterocyclischer Natur sein und gegebenenfalls, z. B. durch Halogenatome, substituiert und/oder ungesättigt sein. Als Beispiele hierfür seien genannt: Bernsteinsäure, Adipinsäure, Korksäure, Azelainsäure, Sebacinsäure, Phthalsäure, Isophthalsäure, Trimellitsäure, Phthalsäureanhydrid, Tetrahydrophthalsäureanhydrid, Hexahydrophthalsäureanhydrid, Tetrachlorphthalsäureanhydrid, Endomethylentetrahydrophthalsäureanhydrid, Glutarsäureanhydrid, Maleinsäure, Maleinsäureanhydrid, Fumarsäure, dimere und trimere Fettsäuren wie Ölsäure, gegebenenfalls in Mischung mit monomeren Fettsäuren, Terephthalsäuredimethylester und Terephthalsäure-bis-glykolester. Als mehrwertige Alkohole kommen z. B. Ethylenglykol, Propylenglykol-(1,2) und -(1,3), Butylenglykol-(1,4) und -(2,3), Hexandiol-(1,6), Octandiol-(1,8), Neopentylglykol, Cyclohexandimethanol (1,4-Bis-hydroxy-methylcyclohexan), 2-Methyl-1,3-propandiol, Glycerin, Trimethylolpropan, Hexantriol-(1,2,6), Butantriol-(1,2,4), Trimethylolethan, ferner Diethylenglykol, Triethylenglykol, Tetraethylenglykol, Polyethylenglykole,

Dipropylenglykol, Polypropylenglykole, Dibutylenglykol und Polybutylenglykole in Frage. Auch Polyester aus Lactonen, z. B. ε-Caprolacton oder Hydroxycarbonsäuren, z. B. ω-Hydroxycapronsäure, sind einsetzbar.

Auch die erfindungsgemäß in Frage kommenden, zwei bis vier, vorzugsweise zwei, Hydroxylgruppen aufweisenden Polyether sind solche der an sich bekannten Art und werden z. B. durch Polymerisation von Epoxiden wie Ethylenoxid, Propylenoxid, Butylenoxid, Tetrahydrofuran, Styroloxid oder Epichlorhydrin mit sich selbst, z. B. in Gegenwart von $BF_3$, oder durch Anlagerung dieser Epoxide, gegebenenfalls im Gemisch oder nacheinander, an Startkomponenten mit reaktionsfähigen Wasserstoffatomen wie Wasser, Alkohole oder Amine, z. B. Ethylenglykol, Propylenglykol-(1,3) oder -(1,2), Trimethylolpropan, 4,4'-Dihydroxydiphenylpropan, Anilin, Ammoniak, Ethanolamin oder Ethylendiamin hergestellt. Vielfach sind solche Polyether bevorzugt, die überwiegend (bis zu 90 Gew.-%, bezogen auf alle vorhandenen OH-Gruppen im Polyether) primäre OH-Gruppen aufweisen. Auch durch Vinylpolymerisate modifizierte Polyether, wie sie z. B. durch Polymerisation von Styrol und Acrylnitril in Gegenwart von Polyethern entstehen (amerikanische Patentschriften 3 383 351, 3 304 273, 3 523 093, 3 110 695, deutsche Patentschrift 1 152 536), sind geeignet.

Unter den Polythioethern seien insbesondere die Kondensationsprodukte von Thiodiglykol mit sich selbst und/oder mit anderen Glykolen, Dicarbonsäuren, Formaldehyd, Aminocarbonsäuren oder Aminoalkoholen angeführt. Je nach den Co-Komponenten handelt es sich bei den Produkten um Polythiomischether, Polythioetherester oder Polythioetheresteramide.

Als Polyacetale kommen z. B. die aus Glykolen, wie Diethylenglykol, Triethylenglykol, 4,4'-Dioxethoxydiphenyldimethylmethan, Hexandiol und Formaldehyd herstellbaren Verbindungen in Frage. Auch durch Polymerisation cyclischer Acetale lassen sich erfindungsgemäß geeignete Polyacetale herstellen.

Als Hydroxylgruppe aufweisende Polycarbonate kommen solche der an sich bekannten Art in Betracht, die z. B. durch Umsetzung von Diolen wie Propandiol-(1,3), Butandiol-(1,4) und/oder Hexandiol-(1,6), Diethylenglykol, Triethylenglykol oder Tetraethylenglykol mit Diarylcarbonaten, z. B. Diphenylcarbonat, oder mit Phosgen hergestellt werden können.

Auch bereits Urethan- oder Harnstoffgruppen enthaltende Polyhydroxylverbindungen sowie gegebenenfalls modifizierte natürliche Polyole wie Rizinusöl sind verwendbar.

Vertreter dieser erfindungsgemäß zu verwendenden Verbindungen sind z. B. in High Polymers, Vol. XVI, »Polyurethanes, Chemistry and Technology«, verfaßt von Saunders-Frisch, Interscience Publishers, New York, London, Band I, 1962, Seiten 32—42 und Seiten 44—54 und Band II, 1964, Seiten 5—6 und 198—199, sowie im Kunststoff-Handbuch, Band VII, Vieweg-Höchtlen, Carl-Hanser-Verlag, München, 1966, z. B. auf den Seiten 45 bis 71 beschrieben.

Selbstverständlich können Mischungen der obengenannten Verbindungen, z. B. Mischungen von Polyethern und Polyestern, eingesetzt werden.

Die den gemachten Ausführungen entsprechenden Polyesterdiole bzw. Polyetherdiole werden bevorzugt eingesetzt.

Zur Herstellung der Isocyanat-Präpolymeren geeignete Diisocyanate der Formel

$$A(NCO)_2$$

sind beispielsweise Tetramethylendiisocyanat, Hexamethylendiisocyanat, 1,12-Dodecandiisocyanat, Cyclobutan-1,3-diisocyanat, Cyclohexan-1,3- und -1,4-diisocyanat sowie beliebige Gemische dieser Isomeren, 1-Isocyanato-3,3,5-trimethyl-5-isocyanatomethyl-cyclohexan, 2,4- und 2,6-Hexahydrotoluylendiisocyanat sowie beliebige Gemische dieser Isomeren, Hexahydro-1,3- und/oder -1,4-phenylendiisocyanat, Perhydro-2,4'- und/oder -4,4'-diphenylmethan-diisocyanat, 1,3- und 1,4-Phenylendiisocyanat, 2,4- und 2,6-Toluylendiisocyanat sowie beliebige Gemische dieser Isomeren, Diphenylmethan-2,4'- und/oder -4,4'-diisocyanat, Naphthylen-1,5-diisocyanat, 2,4'-Diisocyanatodiphenylsulfid, 2-(w-Isocyanatoalkylthio)-phenylisocyanat, Carbodiimidgruppen aufweisende Polyisocyanate, wie sie in der deutschen Patentschrift 1 092 007 beschrieben werden, Diisocyanate, wie sie in der amerikanischen Patentschrift 3 492 330 beschrieben werden. Ferner ist es möglich, beliebige Mischungen dieser Diisocyanate zu verwenden.

Besonders bevorzugt werden in der Regel die technisch leicht zugänglichen Diisocyanate, z. B. das 2,4- und 2,6-Toluylendiisocyanat sowie beliebige Gemische dieser Isomeren (»TDI«), der 2,4'- und/oder 4,4'-Diisocyanatodiphenylmethan eingesetzt.

Gemäß einer bevorzugten Ausführungsform der Variante 2 des erfindungsgemäßen Verfahrens geschieht die Herstellung der NCO-Präpolymeren in situ durch Umsetzung von Polyolen der beispielhaft genannten Art mit einem Überschuß eines als erfindungsgemäßes Ausgangsmaterial einzusetzenden, Schwefel enthaltenden Diisoxyanats, wobei das Diisocyanat in einer solchen über einem NCO/OH-Äquivalentverhältnis von 2 : 1 liegenden Mengenverhältnis eingesetzt wird, daß unmittelbar Lösungen von überschüssigem, Schwefel enthaltendem Diisocyanat in dem sich bildenden NCO-Präpolymer anfallen, deren Gehalt an freiem, Schwefel enthaltendem Diisocyanat den oben gemachten Ausführungen entspricht. Bei dieser Ausführungsform entspricht selbstverständlich

der Rest A des NCO-Präpolymeren der o. g. allgemeinen Formel dem Rest, wie er durch Entfernung der Isocyanatgruppen aus einem Schwefel enthaltenden Ausgangsdiisocyanat der o. g. Formel erhalten wird.

Die erfindungsgemäßen Harnstoffgruppen aufweisenden Diisocyanate stellen entweder bei Raumtemperatur flüssige oder durch einfaches Erwärmen auf maximal 80°C verflüssigbare, bei Raumtemperatur oft pastenartige Substanzen dar. Der Aggregatzustand der erfindungsgemäßen Verbindungen hängt in erster Linie von der Größe des Indices m ab. Im allgemeinen stellen die besonders bevorzugten erfindungsgemäßen Verbindungen mit m = O bei Raumtemperatur flüssige Substanzen dar. Der Wert von m hängt seinerseits vom gewählten Isocyanat/Wasser-Molverhältnis bei der Durchführung des erfindungsgemäßen Verfahrens ab. Bei Verwendung von 0,5 Mol Wasser pro Grammäquivalent an Isocyanatgruppen der erfindungsgemäß einzusetzenden Ausgangsdiisocyanate entstehen in weitgehend selektiver Reaktion derartige erfindungsgemäße Verbindungen mit m = O. Dies ist auf die selektive Reaktionsbereitschaft der Isocyanatgruppen der Ausgangsdiisocyanate zurückzuführen. Falls Wasser nicht im Überschuß angeboten wird, reagiert zunächst die in ortho-Stellung zum Schwefelatom angeordnete, aromatische Isocyanatgruppe ab. Da im Falle der zweiten Ausführungsform des erfindungsgemäßen Verfahrens bei Raumtemperatur flüssige oder durch Erhitzen auf 80°C schmelzbare NCO-Präpolymere eingesetzt werden, handelt es sich auch bei den Lösungen der erfindungsgemäßen, Harnstoffgruppen aufweisenden Diisocyanate in diesen NCO-Präpolymeren um bei Raumtemperatur flüssige oder durch einfaches Erhitzen auf maximal 80°C verflüssigbare Systeme. Derartige Systeme können selbstverständlich auch durch Abmischen entsprechender Mengen an separat hergestellten erfindungsgemäßen, Harnstoffgruppen aufweisenden Diisocyanaten mit den NCO-Präpolymeren erhalten werden.

Der Gehalt der erfindungsgemäßen Verbindungen bzw. ihrer Lösungen in NCO-Präpolymeren an freiem, nicht umgesetztem, Schwefel enthaltendem Diisocyanat liegt bei Verwendung von mindestens 0,5 Mol Wasser pro Grammäquivalent an Isocyanatgruppen der schwefelhaltigen Ausgangsdiisocyanate im allgemeinen unter 0,6 Gew.-%. Grundsätzlich ist es auch möglich, jedoch keineswegs bevorzugt, beim erfindungsgemäßen Verfahren über 80°C schmelzende NCO-Präpolymere einzusetzen, hierbei empfiehlt sich dann insbesondere die Verwendung von wasserabspaltenden Verbindungen wie z. B. von Pinakonhexahydrat anstelle von Wasser, sowie die Mitverwendung von desaktivierenden aciden Verbindungen wie z. B. Phosphorsäure, Toluolsulfonsäure oder Benzylchlorid in Mengen von 0,01 bis 0,1 Gew.-%, bezogen auf Reaktionsgemisch, zur Verhinderung von Nebenreaktionen (Biuretbildung). Auch bei Verwendung von tert.-Butanol als Wasserabspalter sind diese Verbindungen erforderlich, um die Spaltungstemperatur des zunächst entstehenden tert.-Butylurethans herabzusetzen. Bei Verwendung von über 80°C schmelzenden NCO-Präpolymeren werden oft auch durch Erhitzen auf maximal 80°C schmelzbare Lösungen der erfindungsgemäßen Diisocyanate erhalten.

Die literaturbekannten, Diisocyanat-Additionsreaktion beschleunigenden Verbindungen wie tert.-Amine oder metallorganische Verbindungen werden bei der Durchführung des erfindungsgemäßen Verfahrens im allgemeinen nicht mitverwendet, um die Bildung von hochmolekularen, keine Isocyanatgruppen aufweisenden Polyharnstoffen zu verhindern.

Die erfindungsgemäßen, Harnstoffgruppen aufweisenden Diisocyanate stellen besonders wertvolle Ausgangsmaterialien zur Herstellung von Polyurethankunststoffen dar. Einerseits weisen mit ihnen hergestellte Polyurethanelastomere wegen der eingebauten Harnstoff-Segmente besonders interessante mechanische Eigenschaften auf; andererseits handelt es sich bei den erfindungsgemäßen Verbindungen wegen ihrer unterhalb 80°C flüssigen Konsistenz um leicht handhabbare, wegen ihres geringen Dampfdrucks jedoch physiologisch unbedenkliche Substanzen.

Die erfindungsgemäßen Diisocyanate bzw. ihre Lösungen in den genannten NCO-Präpolymeren eignet sich besonders gut zur Herstellung von Polyurethanelastomeren.

Bei der erfindungsgemäßen Verwendung der erfindungsgemäßen, Harnstoffgruppen aufweisenden Diisocyanate zur Herstellung von Polyurethanelastomeren werden beispielsweise die genannten Lösungen der neuen Verbindungen in NCO-Präpolymeren mit Kettenverlängerungsmitteln der an sich bekannten Art auf an sich bekannte Weise zur Umsetzung gebracht. Das Äquivalentverhältnis zwischen Isocyanatgruppen und gegenüber Isocyanatgruppen reaktionsfähigen Gruppen der Kettenverlängerungsmittel liegt hierbei im allgemeinen zwischen 0,9 : 1 und 1,2 : 1, besonders bevorzugt bei 1 : 1 bis 1,1 : 1.

Geeignete Kettenverlängerungsmittel sind beispielsweise Wasser, einfache Glykole des Molekulargewichtsbereichs 62 bis 500 oder organische Diamine mit 2 primären und/oder sekundären Aminogruppen des Molekulargewichtsbereichs 62 bis 500. Auch Hydrazine können als Kettenverlängerungsmittel eingesetzt werden. Geeignete organische Diamine sind beispielsweise aliphatische Diamine wie Ethylendiamin, Hexamethylendiamin, cycloaliphatische Diamine wie 4,4′-Diamino-dicyclohexylmethan oder 1-Methyl-2,4-diamino-cyclohexan oder, besonders bevorzugt, aromatische Diamine wie z. B. Bisanthranilsäureester gemäß den DE-OS 2 040 644 und 2 160 590, 3,5- und 2,4-Diaminobenzoesäureester gemäß DE-OS 2 026 900, die in den DE-OSen 1 803 635, 2 040 650 und 2 160 589 beschriebenen estergruppenhaltigen Diamine, sowie 3,3′-Dichlor-4,4′-diamino-diphenylmethan, 3,3′-Dithioether-4,4′-diaminodiphenylmethan, Phenylendiamine, Toluylendiamine, 3,5-Diethyl-

2,4-diaminotoluol, 4,4'-Diaminodiphenylmethan und 2,2'-Diaminodiphenylsulfid. 2,4'-Diaminodiphenylsulfid oder 2,4'-Diaminodiphenylsulfid genannt.

Geeignete Glykole als Kettenverlängerungsmittel sind z. B. Ethylenglykol Propylenglykol-(1,2) und -(1,3), Butandiol-(1,4) und -(2,3), Petandiol-(1,5), Hexandiol-(1,6), Octandiol-(1,8), Neopentylglykol, 1,4-Bishydroxymethyl-cyclohexan, 2-Methyl-1,3-propandiol, Butendiol, Butindiol, Monochlorhydrin, Glycerin-monoalkyl- oder -monoaryl-ether, Xylylenglykole, das Diels-Alder-Anlagerungsprodukt von Butendiol an Anthracen oder Hexahydrobrenzcatechin.

Bei der Umsetzung der beispielhaft genannten Kettenverlängerungsmittel mit den erfindungsgemäßen Diisocyanaten bzw. deren Lösungen in NCO-Präpolymeren reagieren alle freien NCO-Gruppen glatt ab, was bei Verwendung von festen Harnstoffdiisocyanaten und ihren Suspensionen sehr stark von der Korngröße der Harnstoffdiisocyanate und der Löslichkeit der Umsetzungsprodukte von festem Harnstoffdiisocyanat mit Diol oder Diamin im NCO-Präpolymer abhängt. Selbst kleinste Harnstoffdiisocyanatteilchen zeigen Füllstoffcharakter, da die an den Oberflächen der Teilchen bei Reaktion mit Kettenverlängerern gebildeten Polyurethan- oder Polyharnstoff nicht umgesetztes Harnstoffdiisocyanat verkapseln. Derartiges Füllstoffverhalten ist für Polyurethankunststoffe von großem Nachteil, da ihre mechanischen und dynamischen Eigenschaften stark vermindert und Sollbruchstellen bei Belastung vorgebildet werden. Bei der erfindungsgemäßen Verwendung der neuen Diisocyanate können selbstverständlich die oben, bei der Herstellung der NCO-Präpolymeren beispielhaft genannten höhermolekularen Polyhydroxylverbindungen ebenfalls mitverwendet werden.

Bei der erfindungsgemäßen Verwendung der erfindungsgemäßen neuen Diisocyanate zur Herstellung von Polyurethankunststoffen, insbesondere von Polyurethanelastomeren können auch an sich bekannte Hilfs- und Zusatzmittel wie z. B. Weichmacher, Farb- und Füllstoffe zugegeben werden. Geeignete Weichmacher sind beispielsweise Phthalsäureester und organische Sulfonamide. Besonders günstig sind häufig Schwefel enthaltende Weichmacher wie z. B. Methylen-bis-thioglykolsäurebutylester.

Auch Füllstoffe bzw. Pigmente wie Titandioxid, Siliziumdioxid, Betonit, Calciumsilikat und Ruß finden Verwendung. Sie können direkt in die höhermolekulare Polyhydroxylverbindung oder auch in das NCO-Präpolymer eingearbeitet werden.

Die erfindungsgemäß hergestellten Polyurethan-Elastomeren weisen ein hervorragendes mechanisches Eigenschaftsbild, ein verbessertes Hochtemperaturverhalten und eine ausgezeichnete Beständigkeit gegenüber organischen Lösungsmitteln und Ölen auf. Diese Eigenschaften eröffnen den erfindungsgemäß hergestellten Elastomeren ein breites Einsatzgebiet, beispielsweise als Walzenbeläge, elastische Bauteile für Maschinen, Dichtungen, Puffer, Faltenbälge, Beläge für Kugelmühlen, Schuhsohlen, Zahnräder und Fahrzeugreifen.

Die nachfolgenden Beispiele erläutern die vorliegende Erfindung. Wenn nicht anders vermerkt, sind Mengenangaben als Gewichtsteile bzw. Gewichtsprozente zu verstehen.

Beispielhafte Darstellung von flüssigen, Schwefel enthaltenden Harnstoffdiisocyanaten und deren Lösungen in NCO-Präpolymeren.

## Beispiel 1

Zu 53,6 g (0,2 Mol) 2,4'-Diisocyanatodiphenylsulfid in 200 ml trockenem Aceton werden bei 50 bis 55°C 1,8 g Wasser in 50 ml trockenem Aceton innerhalb 15 Minuten getropft. Die Gasentwicklung tritt augenblicklich ein. Die Mischung wird solange bei 50 bis 55°C gerührt bis kein Gas mehr entsteht. Aceton wird im Vakuum entfernt. Zurück bleibt eine bei Raumtemperatur hochviskose Flüssigkeit der chemischen Zusammensetzung

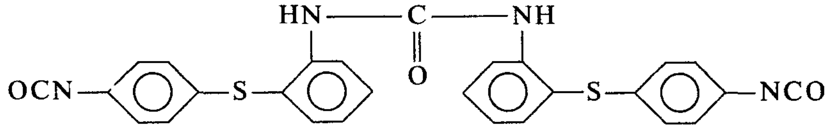

NCO-Gehalt (berechnet) 16,5%
NCO-Gehalt (gefunden) 16,25%

Analog Beispiel 1 wurden folgende Schwefel enthaltenden flüssigen Harnstoffdiisocyanate synthetisiert (vgl. Tabelle).

Tabelle

| Diisocyanat | Harnstoffdiisocyanat | %NCO-Gehalt (ber.) | (gef.) |
|---|---|---|---|
| a) 2.4′-Diisocyanato-3-ethyl-diphenylsulfid | 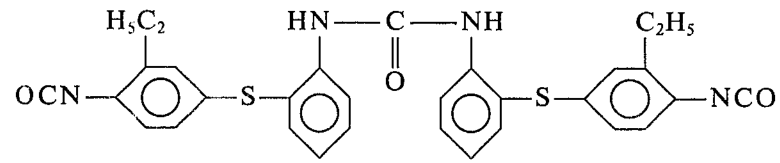 | 14,8 | 14,65 |
| b) 2.4′-Diisocyanato-3′-ethyl-thio-diphenylsulfid | 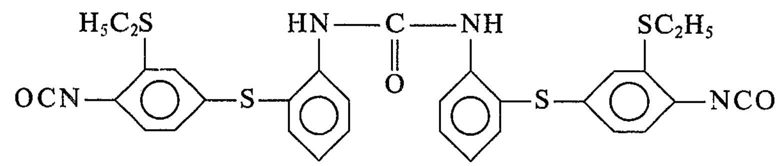 | 13,3 | 13,35 |
| c) 2.4′-Diisocyanato-5-ethyl-diphenylsulfid | 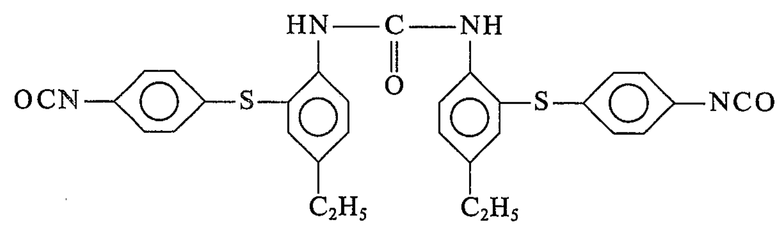 | 14,8 | 14,7 |
| d) 2.4′-Diisocyanato-5-isopropyl-thio-diphenylsulfid | 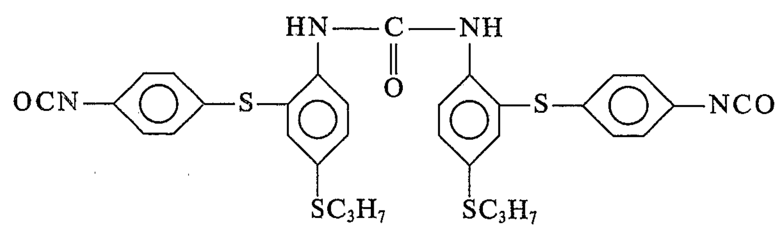 | 12,8 | 12,6 |
| e) 2-(2′-Isocyanatoäthylthio)-phenyl-isocyanat | 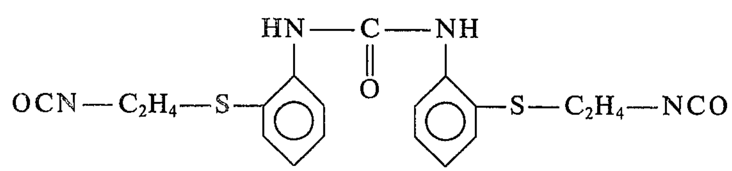 | 20,3 | 20,15 |

Fortsetzung

| Diisocyanat | Harnstoffdiisocyanat | %NCO-Gehalt (ber.) | (gef.) |
|---|---|---|---|
| f) 2-(6′-Isocyanatohexylthio)-phenylisocyanat | 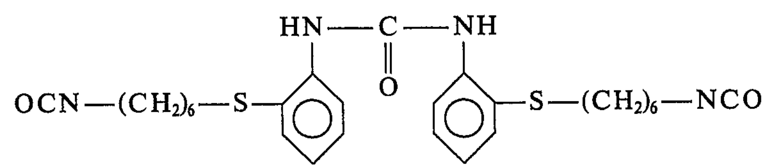 | 16,5 | 16,3 |
| g) 2-(2′-Isocyanatoäthylthio)-4-ethyl-phenylisocyanat | 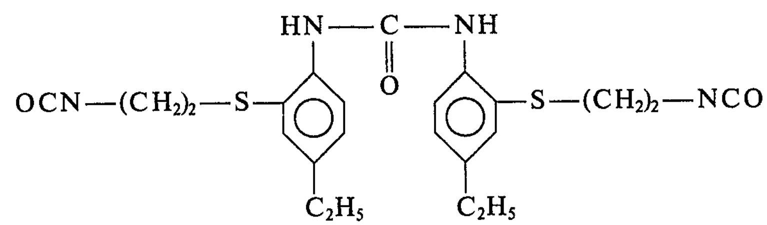 | 17,9 | 17,75 |
| h) 2-(6′-Isocyanatohexylthio)-4-sopropylthic-phenylisocyanat | 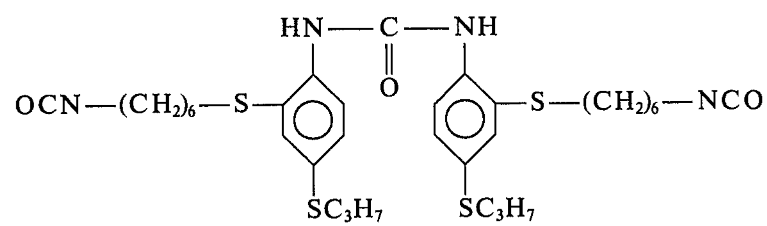 | 12,45 | 12,55 |

Lösungen von Schwefel enthaltenden flüssigen Harnstoffdiisocyanaten in Isocyanato-Präpolymeren.

## Beispiel 2

85% eines NCO-Präpolymeren, vom NCO-Gehalt 3,5%, hergestellt aus einem linearen Polypropylenglykolether vom Mol-Gewicht 2000 (OH-Zahl=56) und 2,4-Toluylendiisocyanat (NCO : OH=2 : 1), werden mit 15% des in Beispiel 1 aufgeführten Harnstoffdiisocyanats bei 60 bis 70°C vermischt. Die gelöste Harnstoffdiisocyanat enthaltende Präpolymerlösung weist einen NCO-Wert von 5,4% NCO auf.

## Beispiel 3

85% eines NCO-Präpolymeren vom NCO-Gehalt 3,4%, hergestellt aus einem Polypropylenglykolether vom Mol-Gewicht 2000 (OH-Zahl=56) und einem technischen Diisocyanatodiphenylmethan (NCO=33,3%) (NCO : OH=2 : 1), werden mit 15% des in der Tabelle aufgeführten Harnstoffdiisocyanats a) bei 60 bis 70°C vermischt. Die Lösung weist einen NCO-Gehalt von 5% NCO auf.

## Beispiel 4

90% eines NCO-Präpolymeren vom NCO-Gehalt 3,5%, hergestellt aus einem linearen Polyesterdiol vom MG=2000 (OH-Zahl=56) auf Basis Adipinsäure und Diethylenglykol und 2,4-Toluylendiisocyanat werden mit 10% des in der Tabelle aufgeführten Harnstoffdiisocyanates e) bei 60 bis 70°C vermischt. Die Lösung weist einen NCO-Gehalt von 5,15% NCO auf.

## Beispiel 5

90% des in Beispiel 4 beschriebenen NCO-Präpolymeren werden bei 60 bis 70°C mit 10% des in der Tabelle aufgeführten flüssigen Harnstoffdiisocyanats f) vermischt. NCO-Gehalt der Lösung=4,8% NCO.

## Beispiel 6

85% des in Beispiel 4 beschriebenen NCO-Präpolymeren werden bei 60 bis 70°C mit 15% des in der Tabelle aufgeführten flüssigen Harnstoffdiisocyanats h) vermischt. NCO-Gehalt der Lösung=4,85% NCO.

## Beispiel 7

90% des in Beispiel 2 beschriebenen NCO-Präpolymeren werden bei 60 bis 70°C mit 10% des in der Tabelle aufgeführten flüssigen Harnstoffdiisocyanats c) vermischt. NCO-Gehalt der Lösung=4,6% NCO.

## Beispiel 8

90% des in Beispiel 3 beschriebenen NCO-Präpolymeren werden bei 60 bis 70°C mit 10% des in der Tabelle aufgeführten flüssigen Harnstoffdiisocyanats b) vermischt. NCO-Gehalt der Lösung=4,4% NCO.

## Beispiel 9

95% eines NCO-Präpolymeren vom NCO-Gehalt 4,9%, hergestellt aus einem Polyetherpolyol-Gemisch aus 45% eines linearen Polypropylenglykols vom MG=2000, 5% eines trifunktionellen Polypropylenglykols vom MG=4800 und 50% eines linearen Polypropylenglykols vom MG=1000 (OH-Zahl=112) und 2,4-Toluylendiisocyanat (NCO/OH=2 : 1) werden mit 5% des in der Tabelle aufgeführten flüssigen Harnstoffdiisocyanats g) vermischt, NCO-Gehalt der Lösung: 5,5% NCO.

## Beispiel 10

90% des in Beispiel 9 beschriebenen Präpolymeren werden bei 60 bis 70°C mit 10% des in der Tabelle aufgeführten flüssigen Harnstoffdiisocyanates d) vermischt. NCO-Gehalt der Lösung = 5,6% NCO.

## Beispiel 11

85% eines NCO-Präpolymeren vom NCO-Gehalt 3,3% hergestellt aus Polypropylenglykoletherdiol gemäß Beispiel 2 und 2,4'-Diisocyanatodiphenylsulfid (NCO/OH = 2 : 1), werden mit 15% des nach Beispiel 1 hergestellten flüssigen Harnstoffdiisocyanats bei 60 bis 70°C vermischt. NCO-Gehalt der Lösung = 5,25% NCO.

## Beispiel 12

Zu 2348 g (1 Mol) eines NCO-Präpolymeren, das aus 2000 g eines linearen Polypropylenglykolethers vom Mol-Gewicht 2000 (OH-Zahl = 56) und 348 g (2,0 Mol) 2,4-Diisocyanatotoluol hergestellt wurde und einen NCO-Gehalt von 3,5% aufweist, werden 268 g (1 Mol) 2,4'-Diisocyanatodiphenylsulfid zugemischt. Zu dieser Mischung werden bei einer Temperatur von 50 bis 60°C innerhalb 1/2 Stunde 9 g (0,5 Mol) Wasser zugesetzt und der Reaktionsansatz nach 5 bis 6 Stunden bei dieser Temperatur gehalten. Nach Beendigung der $CO_2$-Entwicklung (11,5 l) erhält man eine Polyisocyanat-harnstofflösung mit dem Gesamt-NCO-Gehalt von 4,7% NCO.

## Beispiel 13

Das Präpolymerdiisocyanat-Gemisch aus Beispiel 12 wird mit 18,8 g Pinakonhexahydrat vermischt und bei 60 bis 70°C umgesetzt (entsprechend 0,5 Mol Wasser pro Mol freiem 2,4'-Diisocyanatodiphenylsulfid). Man erhält nach 5 bis 6 Stunden bei 60 bis 70°C eine Polyisocyanatharnstoff-Lösung vom Gesamt-NCO-Gehalt 4,5%.

## Beispiel 14

Zu 2500 g (1 Mol) eines NCO-Präpolymeren vom NCO-Gehalt 3,4%, das aus 2000 g eines linearen Polypropylenglykolethers vom Mol-Gewicht 2000 und 500 g eines technischen Diisocyanatodiphenylmethans hergestellt wurde, werden 296 g (1 Mol) 2,4'-Diisocyanato-3'-ethyldiphenylsulfid zugemischt. Zu dieser Mischung werden bei einer Temperatur von 50 bis 60°C innerhalb 1/2 Stunde 9 g (0,5 Mol) Wasser zugesetzt und 5 bis 6 Stunden gerührt. Nach Beendigung der $CO_2$-Entwicklung (11,3 l) erhält man eine Polyisocyanat-harnstofflösung mit dem Gesamt-NCO-Gehalt von 4,5% NCO.

## Beispiel 15

a) Zu 2536 g (1 Mol) eines Präpolymeren vom NCO-Gehalt 3,3% aus einem Polypropylenglykolpolyether vom MG = 2000 und 536 g 2,4'-Diisocyanatodiphenylsulfid werden 268 g (1 Mol) 2,4'-Diisocyanatodiphenylsulfid zugemischt und mit 9 g (0,5 Mol) Wasser bei 50 bis 60°C innerhalb 1/2 Stunde versetzt und 5 bis 6 Stunden gerührt. Nach Beendigung der $CO_2$-Entwicklung erhält man eine Polyisocyanat-harnstofflösung mit dem Gesamt-NCO-Gehalt von 4,42% NCO.

b) 200 g des in a) verwendeten Polyetherdiols werden mit 804 g 2,4'-Diisocyanatodiphenylsulfid präpolymerisiert und dann analog a) mit 9 g Wasser umgesetzt. Man erhält eine Polyisocyanat-harnstofflösung mit dem Gesamt-NCO-Gehalt von 4,35% NCO.

## Beispiel 16

Zu 2336 g (1 Mol) eines Präpolymeren vom NCO-Gehalt 3,6% aus einem Polyesterdiol vom MG = 2000 (OH-Zahl = 56) aus Adipinsäure und Diethylenglykol und 336 g 1,6-Hexamethylendiisocyanat werden mit 220 g (1 Mol) 2-(2'-Isocyanatoethylthio)-phenylisocyanat vermischt, bei 50 bis 60°C mit 9 g Wasser innerhalb 0,5 Stunden versetzt und 6 bis 7 Stunden gerührt. Nach Beendigung der Reaktion beträgt der Gesamt-NCO-Gehalt der Lösung 4,85% NCO.

Beispiel 17

200 g des in Beispiel 16 verwendeten Polyesterdiols werden mit 105 g 2-(6'-Isocyanatohexylthio)-4-isopropylthio-phenylisocyanat präpolymerisiert und mit 0,9 g Wasser bei 50 bis 60° C versetzt und 6 bis 7 Stunden gerührt. Nach Beendigung der Reaktion beträgt der Gesamt-NCO-Gehalt der Lösung 4% NCO.

Beispiel 18

Zu 253,6 g (0,1 Mol) des Präpolymeren aus Beispiel 15a) werden 26,8 g (0,1 Mol) 2,4'-Diisocyanatodiphenylsulfid zugemischt, 1,1 g Wasser (0,61 Mol) langsam bei 50 bis 60°C zugetropft und 5 bis 6 Stunden gerührt. Der Gesamt-NCO-Gehalt beträgt 4,15% NCO.

Beispiel 19

Zu 255,2 g (0,1 Mol) eines Präpolymeren vom NCO-Gehalt 3,2% aus einem Polyesterdiol vom MG = 2000, OH-Zahl = 56, und 55,2 g (0,2 Mol) 2-(6'-Isocyanatohexylthio)-phenylisocyanat werden 22,0 g (0,1 Mol) 2-(2'-Isocyanatoethylthio)-phenylisocyanat zugemischt, 1,3 g (0,72 Mol) Wasser langsam bei 50 bis 60° C zugetropft und 5 bis 6 Stunden gerührt. Der Gesamt-NCO-Gehalt beträgt 3,7% NCO.

Beispiel 20

a) Zu 244,0 g (0,1 Mol) eines Präpolymeren vom NCO-Gehalt 3,4% NCO aus einem Polyesterdiol vom MG = 2000, OH-Zahl = 2000, OH-Zahl = 56, und 44,0 g (0,2 Mol) 2-(2'-Isocyanatoethylthio)-phenylisocyanat werden 33 g (0,15 Mol) 2-(2'-Isocyanatoethylthio)-phenylisocyanat zugemischt, 1,8 g Wasser (0,1 Mol) bei 50 bis 60°C in 45 Minuten zugetropft und 6 bis 7 Stunden gerührt. Der Gesamt-NCO-Gehalt beträgt 4,5% NCO.
b) 200,0 g des in a) verwendeten Polyesterdiols werden mit 77 g 2-(2'-Isocanatoethylthio)-phenylisocyanat vermischt und präpolymerisiert. Der NCO-Gehalt des Präpolymeren beträgt 10,6% NCO. Bei 50 bis 60°C werden 1,8 g (0,1 Mol) Wasser wie in a) beschrieben zugesetzt. Der Gesamt-NCO-Gehalt beträgt 4,4% NCO.

Elastomerbeispiele

Beispiel 21

a) 100 g der im Beispiel 2 hergestellten Polyisocyanat-harnstofflösung mit einem NCO-Gehalt von 5,4% werden bei 60 bis 80° C im Vakuum entgast und mit 14,17 g 4-Chlor-3,5-diamino-benzoesäure-isobutylester innerhalb von 30 Sekunden verrührt. (NCO = $NH_2$ = 1,1 : 1). Der Reaktionsansatz wird dann in eine 120°C heiße Metallform gegossen. Die Gießzeit beträgt ca. 5 Minuten. Nach etwa 12 Minuten kann der Gießling entfernt werden. Nach Temperung von jeweils 24 Stunden bei 120° C wurden die mechanischen Eigenschaften des Elastomeren bestimmt.
b) Analog mit 10,4 g 3,5-Diethyl-2,4-diaminotoluol

| | a) | b) |
|---|---|---|
| Zugfestigkeit (DIN 53 504) | 34,3 mPa | 32,1 mPa |
| Bruchdehnung (DIN 53 504) | 644% | 670% |
| Weiterreißfestigkeit (DIN 53 515) | 35,7 KN/m | 35 KN/m |
| Shore Härte A (DIN 53 505) | 85 | 83 |
| Elastizität (DIN 53 512) | 55% | 53% |

### Beispiel 22

100 g der im Beispiel 4 hergestellten Polyisocyanato-harnstofflösung werden bei 80 bis 100°C im Vakuum entgast und anschließend mit 13,5 g 3,5-Diamino-4-chlorbenzoesäureisobutylester innerhalb von 30 Sekunden verrührt. Das $NCO/NH_2$-Molverhältnis = 1,1 : 1. Der Reaktionsansatz wird in eine auf 100°C erwärmte Form gegossen. Nach einer Gießzeit von 3,5 Minuten erhält man nach einer Temperzeit von etwa 10 Stunden bei 120 bis 130°C einen Formkörper mit untenstehenden Eigenschaften.

| | |
|---|---|
| Shore Härte A (DIN 53505) | 75,0 |
| Zugfestigkeit (MPa) | 20,5 |
| Weiterreißfestigkeit (KN/m) | 31,9 |
| Elastizität (%) | 48 |

### Beispiel 23

a) 100 g der im Beispiel 10 genannten Polyisocyanat-harnstofflösung mit einem NCO-Gehalt von 5,6% werden nach dem Entgasen bei 80°C mit 10,8 g 3,5-Diethyl-2,4-diaminotoluol verrührt. Nach einer Gießzeit von 1 Minute und einer Verfestigungszeit von 10 Minuten erhält man einen elastischen Formkörper, der weitere 24 Stunden bei 120°C ausgeheizt wird und danach folgende mechanische Werte aufweist.

b) Analog a) mit 14,4 g 4-Chlor-3,5-diamino-benzoesäureisobutylester

| | a) | b) |
|---|---|---|
| Zugfestigkeit (mPa) | 38,5 | 37,6 |
| Bruchdehnung (%) | 480 | 510 |
| Weiterreißwiderstand (KN/m) | 43,3 | 46,8 |
| Shore Härte A | 88 | 90 |
| Elastizität (%) | 51 | 52 |

### Beispiel 24

100 g der in Beispiel 13 hergestellten Polyisocyanat-harnstofflösung werden bei 80°C entgast und mit 11,8 g 4-Chlor-3,5-diaminobenzoesäureisobutylester innerhalb 30 Sekunden verrührt. ($NCO = NH_2$ = 1,1 : 1). Der Reaktionsansatz wird in eine auf 100°C vorgewärmte Form gegossen. Die Gießzeit beträgt 5 Minuten. Nach 15 Minuten kann der Gießling entformt werden. Nach einer Temperzeit von 24 Stunden bei 110°C werden folgende mechanische Eigenschaften des Elastomeren erhalten:

| | |
|---|---|
| Zugfestigkeit DIN 53504 | 17,5 MPa |
| Bruchdehnung DIN 53504 | 638% |
| Weiterreißwiderstand DIN 53515 | 24,2 KN/m |
| Shore A DIN 53505 | 78 |
| Elastizität DIN 53512 | 50% |

### Beispiel 25

100 g der in Beispiel 14 hergestellten Polyisocyanat-harnstofflösung werden mit 11,8 g 4-Chlor-3,5-diaminobenzoesäureisobutylester wie in Beispiel 24 umgesetzt. Die Gießzeit beträgt 4,5 Minuten, die Entformungszeit 16 Minuten. Mechanische Eigenschaften:

| | |
|---|---|
| Zugfestigkeit DIN 53504 | 19,7 MPa |
| Bruchdehnung DIN 53504 | 620% |
| Weiterreißwiderstand DIN 53515 | 28,2 KN/m |
| Shore A DIN 53505 | 85 |
| Elastizität DIN 53512 | 51% |

Beispiel 26

100 g der in Beispiel 15a) hergestellten Polyisocyanat-harnstofflösung vom NCO-Gehalt 4,42% werden bei 100°C mit 11,6 4-Chlor-3,5-diamino-benzoesäureisobutylester innerhalb 30 Sekunden vermischt (NCO = $NH_2$ = 1,1 : 1) und in eine auf 110°C vorgewärmte Form gegossen. Die Mischung bleibt 4 Minuten gießbar. Der Gießling kann nach 13 Minuten entformt werden. Nach einer Temperzeit von 24 Stunden bei 110°C werden folgende mechanischen Werte erhalten:

| | |
|---|---|
| Zugfestigkeit DIN 53504 | 19,3 MPa |
| Bruchdehnung DIN 53504 | 625% |
| Weiterreißwiderstand DIN 53515 | 28,5 KN/m |
| Shore A DIN 53505 | 77 |
| Elastizität DIN 53512 | 49% |

Beispiel 27

Jeweils 100 g der in Beispiel 20 a) und b) hergestellten Polyisocyanat-harnstofflösungen vom NCO-Gehalt 4,5 bzw. 4,4% NCO werden bei 100°C mit 11,8 g bzw. 11,55 g 4-Chlor-3,5-diaminobenzoesäureisobutylester analog Beispiel 26 umgesetzt. (NCO = $NH_2$ = 1,1 : 1). Die Gießzeit beträgt 6 Minuten, die Entformungszeit 16 Minuten. Mechanische Eigenschaften:

| | Lösung 20a) | Lösung 20b) |
|---|---|---|
| Zugfestigkeit DIN 53 504 | 14,7 mPa | 14,8 mPa |
| Bruchdehnung DIN 53 504 | 825% | 850% |
| Weiterreißwiderstand DIN 53 515 | 21,4 KN/m | 22,4 KN/m |
| Shore A DIN 53 505 | 72 | 73 |
| Elastizität DIN 53 512 | 52% | 50% |

**Patentansprüche für die Vertragsstaaten: BE, DE, FR, GB, IT, NL**

1. Bei Raumtemperatur flüssige oder durch Erhitzen auf maximal 80°C verflüssigbare, gegebenenfalls als bei Raumtemperatur flüssige oder durch Erhitzen auf maximal 80°C verflüssigbare, 5- bis 50gew.-%ige Lösung in bei Raumtemperatur flüssigen oder durch Erhitzen auf maximal 80°C verflüssigbarem NCO-Präpolymeren der Formel

$$D-(OCO-NH-A-NCO)_n$$

vorliegende, Harnstoffgruppen aufweisende Diisocyanate der Formel

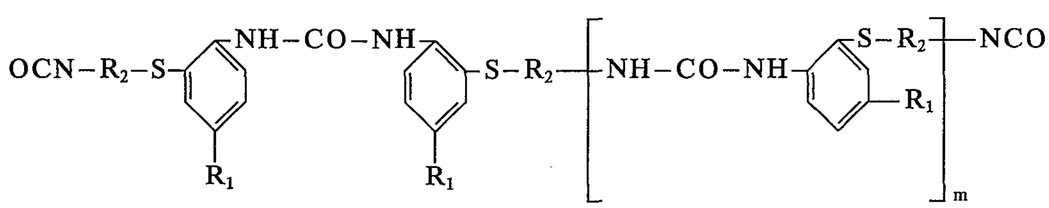

wobei

m für O oder eine ganze oder (im statistischen Mittel) gebrochene Zahl von 0 bis 3 steht,
n für eine ganze oder (im statistischen Mittel) gebrochene Zahl von 2 bis 4 steht,
A für einen Rest steht, wie er durch Entfernung der Isocyanatgruppen aus einem organischen Diisocyanat erhalten wird,
D für einen Rest steht, wie er durch Entfernung der Hydroxylgruppen aus einer n-funktionellen Polyhydroxylverbindung des Molekulargewichtsbereichs 500 bis 8000 bzw. durch Entfernung der Hydroxylgruppen aus einem Gemisch derartiger Polyhydroxylverbindungen erhalten wird,

$R_1$ Wasserstoff, eine Alkylgruppe mit 1 bis 4 Kohlenstoffatomen oder eine Alkylthiogruppe mit 1 bis 4 Kohlenstoffatomen steht,

$R_2$ für einen, gegebenenfalls durch Alkylgruppen mit 1 bis 4 Kohlenstoffatomen oder durch eine Alkylthiogruppe mit 1 bis 4 Kohlenstoffatomen substituierten Phenylenrest oder einen linearen oder verzweigten aliphatischen Kohlenwasserstoffrest mit 2 bis 12 Kohlenstoffatomen steht, wobei zwischen dem Stickstoffatom und dem Schwefelatom mindestens 2 Kohlenstoffatome angeordnet sind.

2. Bei Raumtemperatur flüssige Diisocyanate gemäß Anspruch 1 der Formel

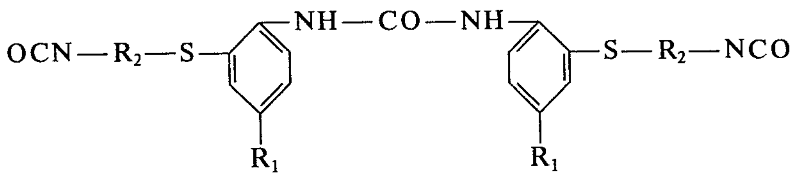

wobei
$R_1$ und $R_2$ die in Anspruch 1 genannte Bedeutung haben.

3. Verfahren zur Herstellung von Diisocyanaten bzw. Diisocyanat-Lösungen in NCO-Präpolymeren gemäß Anspruch 1 und 2, dadurch gekennzeichnet, daß man Diisocyanate der Formel

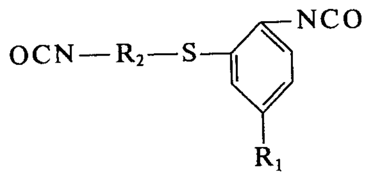

mit 0,4 bis 0,8 Mol pro Mol Diisocyanat Wasser oder einer entsprechenden Menge einer wasserabspaltenden Verbindung zur Reaktion bringt und gegebenenfalls das so erhaltene Harnstoffgruppen aufweisende Diisocyanat anschließend in 50 bis 95 Gew.-%, bezogen auf Gesamtgemisch, eines NCO-Präpolymeren der in Anspruch 1 genannten Art löst.

4. Verfahren gemäß Anspruch 3, dadurch gekennzeichnet, daß man die Umsetzung zwischen dem Ausgangsdiisocyanat und dem Wasser in Gegenwart von 50 bis 95 Gew.-%, bezogen auf Gesamtgemisch, eines NCO-Präpolymeren der in Anspruch 1 genannten Art durchführt.

5. Verwendung der Harnstoffgruppen aufweisenden Diisocyanate bzw. ihre Lösungen in NCO-Präpolymeren gemäß Anspruch 1 und 2 als Aufbaukomponente bei der Herstellung von Polyurethankunststoffen nach dem Isocyanat-Polyadditionsverfahren.

**Patentansprüche für den Vertragsstaat: AT**

1. Verfahren zur Herstellung von bei Raumtemperatur flüssigen oder durch Erhitzen auf maximal 80°C verflüssigbaren, gegebenenfalls als bei Raumtemperatur flüssige oder durch Erhitzen auf maximal 80°C verflüssigbare, 5- bis 50gew.-%ige Lösung in bei Raumtemperatur flüssigen oder durch Erhitzen auf maximal 80°C verflüssigbaren NCO-Präpolymeren der Formel

$$D\text{—}(OCO\text{—}NH\text{—}A\text{—}NCO)_n$$

vorliegenden, Harnstoffgruppen aufweisenden Diisocyanaten der Formel

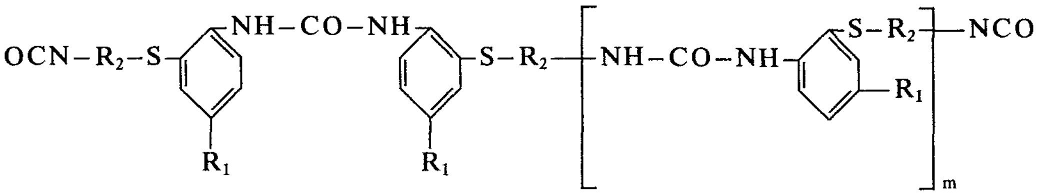

wobei

m für 0 oder eine ganze oder (im statistischen Mittel) gebrochene Zahl von 0 bis 3 steht,

n für eine ganze oder (im statistischen Mittel) gebrochene Zahl von 2 bis 4 steht,

A für einen Rest steht, wie er durch Entfernung der Isocyanatgruppen aus einem organischen Diisocyanat erhalten wird,

D für einen Rest steht, wie er durch Entfernung der Hydroxylgruppen aus einer n-funktionellen Polyhydroxylverbindung des Molekulargewichtsbereichs 500 bis 8000 bzw. durch Entfernung der

Hydroxylgruppen aus einem Gemisch derartiger Polyhydroxylverbindungen erhalten wird,

$R_1$ Wasserstoff, eine Alkylgruppe mit 1 bis 4 Kohlenstoffatomen oder eine Alkylthiogruppe mit 1 bis 4 Kohlenstoffatomen steht,

$R_2$ für einen, gegebenenfalls durch Alkylgruppen mit 1 bis 4 Kohlenstoffatomen oder durch eine Alkylthiogruppe mit 1 bis 4 Kohlenstoffatomen substituierten Phenylenrest oder einen linearen oder verzweigten aliphatischen Kohlenwasserstoffrest mit 2 bis 12 Kohlenstoffatomen steht, wobei zwischen dem Stickstoffatom und dem Schwefelatom mindestens 2 Kohlenstoffatome angeordnet sind,

dadurch gekennzeichnet, daß man Diisocyanate der Formel

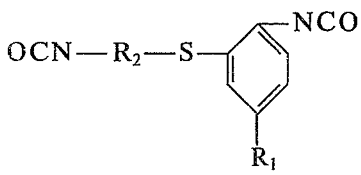

mit 0,4 bis 0,8 Mol pro Mol Diisocyanat Wasser oder einer entsprechenden Menge einer wasserabspaltenden Verbindung zur Reaktion bringt und gegebenenfalls das so erhaltene Harnstoffgruppen aufweisende Diisocyanat anschließend in 50 bis 95 Gew.-%, bezogen auf Gesamtgemisch, eines NCO-Präpolymeren der genannten Art löst.

2. Verfahren gemäß Anspruch 1, dadurch gekennzeichnet, daß man die Umsetzung zwischen dem Ausgangsdiisocyanat und dem Wasser in Gegenwart von 50 bis 95 Gew.-%, bezogen auf Gesamtgemisch, eines NCO-Präpolymeren der in Anspruch 1 genannten Art durchführt.

3. Verwendung der gemäß Anspruch 1 und 2 erhältlichen Harnstoffgruppen aufweisenden Diisocyanate bzw. ihrer Lösungen in NCO-Präpolymeren als Aufbaukomponente bei der Herstellung von Polyurethankunststoffen nach dem Isocyanat-Polyadditionsverfahren.

**Claims for the contracting states: BE, DE, FR, GB, IT, NL**

1. Diisocyanates containing urea groups, which diisocyanates are liquid at room temperature or can be liquified by heating to a temperature of not more than 80° C, optionally in the form of solutions of from 5 to 50% by weight which are liquid at room temperature or which can be liquified by heating to a temperature of not more than 80° C, in isocyanate prepolymers of the formula:

$$D-(OCO-NH-A-NCO)_n$$

which are liquid at room temperature or which can be liquified by heating to a temperature of not more than 80°C, which diisocyanates correspond to the formula

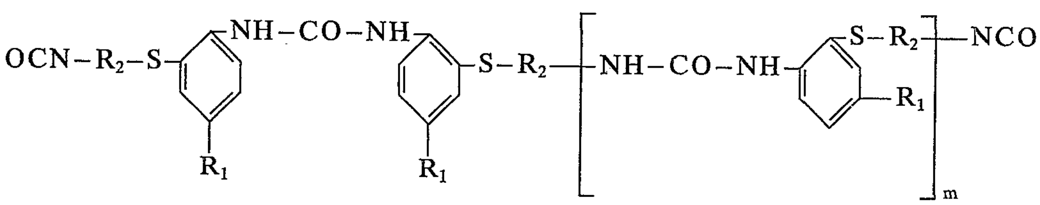

wherein

m represents 0 or a number of from 0 to 3 which is whole or fractional (on statistical average),

n represents a whole or fractional number (on statistical average) of from 2 to 4,

A represents a residue such as is obtained by the removal of the isocyanate groups from an organic diisocyanate,

D represents a residue such as is obtained by the removal of the hydroxyl groups from an n-functional polyhydroxyl compound of the molecular weight range of from 500 to 8,000 or by the removal of the hydroxyl groups from a mixture of such polyhydroxyl compounds,

$R_1$ represents hydrogen, an alkyl group having from 1 to 4 carbon atoms or an alkylthio group having from 1 to 4 carbon atoms.

$R_2$ represents a phenylene group optionally substituted by alkyl groups having from 1 to 4 carbon atoms or by an alkylthio group having from 1 to 4 carbon atoms or it represents a linear or branched chain aliphatic hydrocarbon group having from 2 to 12 carbon atoms, with at least 2 carbon atoms being situated between the nitrogen atom and the sulphur atom.

2. Diisocyanates which are liquid at room temperature according to claim 1, of the formula

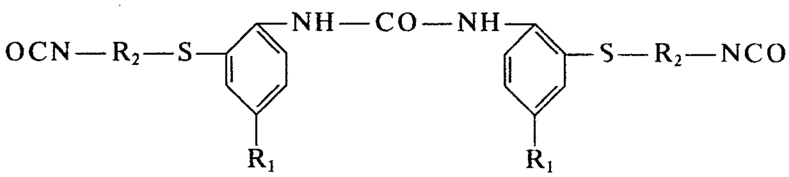

wherein
$R_1$ and $R_2$ have the meaning indicated in Claim 1.

3. A process for the preparation of diisocyanates or solutions of diisocyanates in isocyanate prepolymers according to claims 1 and 2, characterised in that diisocyanates of the formula

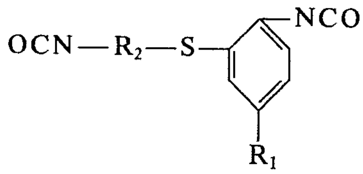

are reacted with from 0.4 to 0.8 mol of water per mol of diisocyanate, or with a corresponding quantity of a compound from which water is split off, and the resulting diisocyanate containing urea groups is optionally thereafter dissolved in from 50 to 95% by weight, based on the total mixture, of an isocyanate prepolymer of the type mentioned in claim 1.

4. A process according to Claim 3, characterised in that the reaction between the diisocyanate used as a starting material and water is carried out in the presence of from 50 to 95% by weight, based on the total mixture, of an isocyanate prepolymer of the type mentioned in claim 1.

5. The use of the diisocyanates containing urea groups or their solutions in isocyanate prepolymers according to claims 1 and 2 as components for the synthesis of polyurethanes by the isocyanate polyaddition process.

**Claims for the contracting state: AT**

1. Process for the preparation of diisocyanates containing urea groups, which diisocyanates are liquid at room temperature or can be liquified by heating to a temperature of not more than 80°C, optionally in the form of solutions of from 5 to 50% by weight which are liquid at room temperature or which can be liquified by heating to a temperature of not more than 80° C, in isocyanate prepolymers of the formula:

$$D{-}(OCO{-}NH{-}A{-}NCO)_n$$

which are liquid at room temperature or which can be liquified by heating to a temperature of not more than 80°C, which diisocyanates correspond to the formula

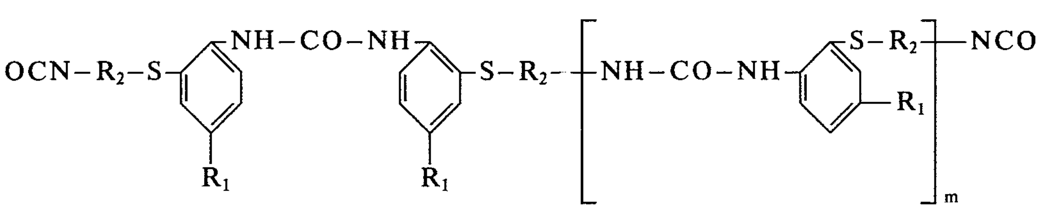

wherein

m represents 0 or a number of from 0 to 3 which is whole or fractional (on statistical average),
n represents a whole or fractional number (on statistical average) of from 2 to 4,
A represents a residue such as is obtained by the removal of the isocyanate groups from an organic diisocyanate,
D represents a residue such as is obtained by the removal of the hydroxyl groups from an n-functional polyhydroxyl compound of the molecular weight range of from 500 to 8,000 or by the removal of the hydroxyl groups from a mixture of such polyhydroxyl compounds,
$R_1$ represents hydrogen, an alkyl group having from 1 to 4 carbon atoms or an alkylthio group having from 1 to 4 carbon atoms.
$R_2$ represents a phenylene group optionally substituted by alkyl groups having from 1 to 4 carbon atoms or by an alkylthio group having from 1 to 4 carbon atoms or it represents a linear or

branched chain aliphatic hydrocarbon group having from 2 to 12 carbon atoms, with at least 2 carbon atoms being situated between the nitrogen atom and the sulphur atom,

characterised in that diisocyanates of the formula

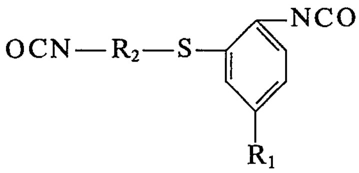

are reacted with from 0.4 to 0.8 mol of water per mol of diisocyanate, or with a corresponding quantity of a compound from which water is split off, and the resulting diisocyanate containing urea groups is optionally thereafter dissolved in from 50 to 95% by weight, based on the total mixture, of an isocyanate prepolymer of the type mentioned.

2. A process according to Claim 1, characterised in that the reaction between the diisocyanate used as a starting material and water is carried out in the presence of from 50 to 95% by weight, based on the total mixture, of an isocyanate prepolymer of the type mentioned in claim 1.

3. The use of the diisocyanates containing urea groups or their solutions in isocyanate prepolymers, obtainable according to claims 1 and 2, as components for the synthesis of polyurethanes by the isocyanate polyaddition process.

**Revendications pour les états contractants: BE, DE, FR, GB, IT, NL**

1. Diisocyanates porteurs de groupes urée, liquides à la température ambiante ou liquéfiables par chauffage à 80° C au maximum, se présentant éventuellement sous la forme de solution à 5—50% en poids, liquides à la température ambiante ou liquéfiables par chauffage à un maximum de 80° C, dans des prépolymères NCO de formule

$$D\text{—}(OCO\text{—}NH\text{—}A\text{—}NCO)_n$$

liquides à la température ambiante ou liquéfiables par chauffage à un maximum de 80°C, de formule

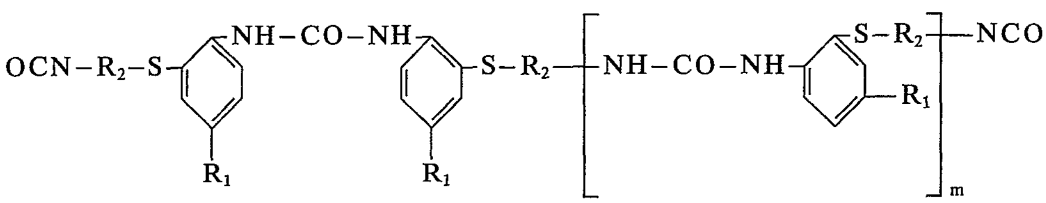

formules dans lesquelles

- m est égal à 0 ou à un nombre entier ou fractionnaire (en moyenne statistique) de 0 à 3,
- n est un nombre entier ou un nombre fractionnaire (en moyenne statistique) de 2 à 4,
- A représente un reste tel qu'on en obtient par élimination des groupes isocyanate d'un diisocyanate organique,
- D est un reste tel qu'on en obtient en éliminant les groupes hydroxyle d'un composé polyhydroxylique n-fonctionnel de poids moléculaire compris dans la plage de 500 à 8000 ou en éliminant les groupes hydroxyle d'un mélange de tels composés polyhydroxyliques,
- $R_1$ représente l'hydrogène, un groupe alkyle ayant 1 à 4 atomes de carbone ou un groupe alkylthio ayant 1 à 4 atomes de carbone,
- $R_2$ représente un reste phénylène éventuellement substitué par des groupes alkyle ayant 1 à 4 atomes de carbone ou par un groupe alkylthio ayant 1 à 4 atomes de carbone, ou un reste hydrocarboné aliphatique linéaire ou ramifié ayant 2 à 12 atomes de carbone, au moins 2 atomes de carbone étant disposés entre l'atome d'azote et l'atome de soufre.

2. Diisocyanates liquides à la température ambiante suivant la revendication 1, de formule

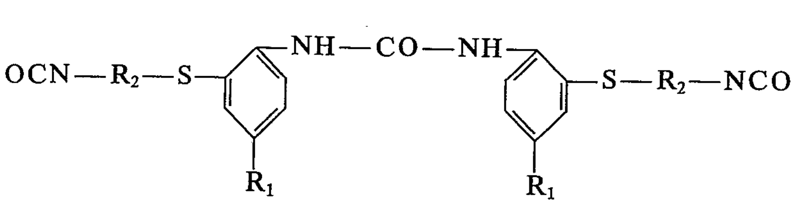

dans laquelle

$R_1$ et $R_2$ ont la définition indiquée dans la revendication 1.

3. Procédé de production de diisocyanates et de solutions de diisocyanates dans des prépolymères NCO suivant les revendications 1 et 2 caractérisé en ce qu'on fait réagir des diisocyanates de formule

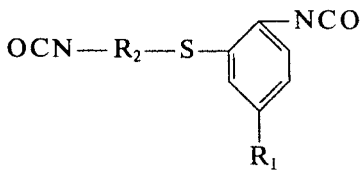

avec 0,4 à 0,8 mole, par mole de diisocyanate, d'eau ou une quantité correspondante d'un composé cédant de l'eau et, le cas échéant, on dissout ensuite le diisocyanate porteur de groupes urée ainsi obtenu dans 50 à 95% en poids, par rapport au mélange total, d'un prépolymère NCO du type mentionné dans la revendication 1.

4. Procédé suivant la revendication 3, caractérisé en ce qu'on conduit la réaction entre le diisocyanate de départ et l'eau en présence de 50 à 95% en poids, par rapport au mélange total, d'un prépolymère NCO du type mentionné dans la revendication 1.

5. Utilisation des diisocyanates porteurs de groupes urée et de leurs solutions dans des prépolymères NCO suivant les revendications 1 et 2 comme composants structuraux dans la production de matières plastiques du type polyuréthanne selon le procédé de polyaddition d'un isocyanate.

**Revendications pour l'état contractant: AT**

1. Procédé de production de diisocyanates porteurs de groupes urée, liquides à la température ambiante ou liquéfiables par chauffage à 80° C au maximum, se présentant éventuellement sous la forme de solution à 5—50% en poids, liquide à la température ambiante ou liquéfiable par chauffage à un maximum de 80° C, dans des prépolymères NCO de formule

$$D{-}\!(OCO{-}NH{-}A{-}NCO)_n$$

liquides à la température ambiante ou liquéfiables par chauffage à un maximum de 80°C, de formule

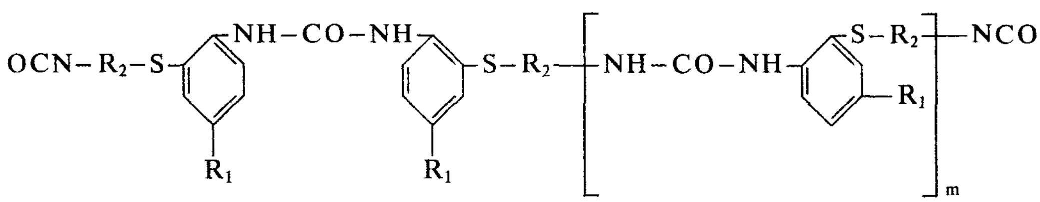

formules dans lesquelles

- m est égal à 0 ou à un nombre entier ou fractionnaire (en moyenne statistique) de 0 à 3,
- n est un nombre entier ou un nombre fractionnaire (en moyenne statistique) de 2 à 4,
- A représente un reste tel qu'on en obtient par élimination des groupes isocyanate d'un diisocyanate organique,
- D est un reste tel qu'on en obtient en éliminant les groupes hydroxyle d'un composé polyhydroxylique n-fonctionnel de poids moléculaire compris dans la plage de 500 à 8000 ou en éliminant les groupes hydroxyle d'un mélange de tels composés polyhydroxyliques,
- $R_1$ représente l'hydrogène, un groupe alkyle ayant 1 à 4 atomes de carbone ou un groupe alkylthio ayant 1 à 4 atomes de carbone,
- $R_2$ représente un reste phénylène éventuellement substitué par des groupes alkyle ayant 1 à 4 atomes de carbone ou par un groupe alkylthio ayant 1 à 4 atomes de carbone, ou un reste hydrocarboné aliphatique linéaire ou ramifié ayant 2 à 12 atomes de carbone, au moins 2 atomes de carbone étant disposés entre l'atome d'azote et l'atome de soufre.

carctérisé en ce qu'on fait réagir des diisocyanatws de formule

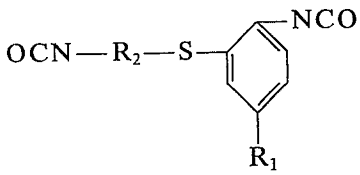

avec 0,4 à 0,8 mole, par mole de diisocyanate, d'eau ou une quantité correspondante d'un composé cédant de l'eau et, le cas échéant, on dissout ensuite le diisocyanate porteur de groupes urée ainsi obtenu dans 50 à 95% en poids, par rapport au mélange total, d'un prépolymère NCO du type mentionné.

2. Procédé suivant la revendication 1, caractérisé en ce qu'on conduit la réaction entre le diisocyanate de départ et l'eau en présence de 50 à 95% en poids, par rapport au mélange total, d'un prépolymère NCO du type mentionné dans la revendication 1.

3. Utilisation des diisocyanates porteurs de groupes urée ou de leurs solution dans des prépolymères NCO, obtenus conformément aux revendications 1 et 2, comme composants structuraux dans la production de matières plastiques du type polyuréthanne par le procédé de polyaddition d'un isocyanate.